# EUROPEAN PATENT APPLICATION

(11) **EP 2 047 875 A2**
(43) Date of publication of application: **15.04.2009**
(21) Application number: 08253306.8
(22) Date of filing: 10.10.2008
(51) Int. Cl.: A61M 5/145, A61M 5/315, A61M 5/50, A61M 5/24

(54) **Drug delivery system with breakaway plunger extractor**

(30) Priority: 11.10.2007 US 870635
(71) Applicant: Animas Corporation, West Chester, PA 19380 (US)
(72) Inventor: Lawson, William, Trappe, PA 19426 (US)
(74) Representative: Tunstall, Christopher Stephen

(57) **Abstract**

Described is a drug delivery system (200) and method for inserting a cartridge (102) into a chamber (202) in a drug infusion device (200) to inhibit refilling or reuse of a cartridge. In one embodiment, a drug delivery system (200) is provided that includes a chamber (202) and a cartridge (102) configured for insertion into the chamber. The cartridge includes a hollow barrel (112) for receiving and storing a drug, a plunger (100) having a distal end (106) and a proximal end (104), where the distal end of the plunger is slidably insertable and movable within the barrel (112). A breakaway extractor (110) is disposed external to the barrel (112) and is removably attached to the proximal end (104) of the plunger (100). After insertion of the plunger (100) into the barrel (112), the breakaway extractor (110) may be detached from the cartridge (102), to inhibit the retraction of the plunger from within the barrel of the cartridge and refilling of the cartridge.

## Description

### FIELD OF THE INVENTION

The present invention relates, in general, to cartridges used in drug delivery devices and, more particularly, to cartridges with breakaway plungers and methods for their use.

### BACKGROUND OF THE INVENTION

The use of drug delivery devices for various types of drug therapy is becoming more common as the automated infusion of a drug may provide more reliable and more precise treatment to a patient.

Diabetes is a major health concern, as it can significantly impede on the freedom of action and lifestyle of persons afflicted with this disease. Typically, treatment of the more severe form of the condition, Type I (insulin-dependent) diabetes, requires one or more insulin injections per day, referred to as multiple daily injections. Insulin is required to control glucose or sugar in the blood, thereby preventing hyperglycemia which, if left uncorrected, can lead to ketosis. Additionally, improper administration of insulin therapy can result in hypoglycemic episodes, which can cause coma and death. Hyperglycemia in diabetics has been correlated with several long-term effects of diabetes, such as heart disease, atherosclerosis, blindness, stroke, hypertension, and kidney failure.

The value of frequent monitoring of blood glucose as a means to avoid or at least minimize the complications of Type I diabetes is well established. Patients with Type II (non-insulin-dependent) diabetes can also benefit from blood glucose monitoring in the control of their condition by way of diet and exercise. Thus, careful monitoring of blood glucose levels and the ability to accurately and conveniently infuse insulin into the body in a timely manner is a critical component in diabetes care and treatment.

In order to more effectively control diabetes in a manner that reduces the limitations imposed by this disease on the lifestyle of the affected person, various devices for facilitating blood glucose (BG) monitoring have been introduced. Typically, such devices, or meters, permit the patient to quickly, and with a minimal amount of physical discomfort, obtain a sample of their blood or interstitial fluid which is then analyzed by the meter. In most cases, the meter has a display screen which shows the BG reading for the patient. The patient may then dose themselves with the appropriate amount, or bolus, of insulin. For many diabetics, this results in having to receive multiple daily injections of insulin. In many cases, these injections are self administered.

Due to the debilitating effects that abnormal BG levels can have on patients, i.e., hyperglycemia, persons experiencing certain symptoms of diabetes may not be in a situation where they can safely and accurately self-administer a bolus of insulin. Moreover, persons with active lifestyles find it extremely inconvenient and imposing to have to use multiple daily injections of insulin to control their blood sugar levels, as this may interfere or prohibit their ability to engage in certain activities. For others with diabetes, multiple daily injections may simply not be the most effective means for controlling their BG levels. Thus, to further improve both accuracy and convenience for the patient, insulin infusion pumps have been developed.

Insulin pumps are generally devices which are worn on the patient's body, either above or below their clothing. These relatively small, unobtrusive devices typically store a quantity of insulin in a replaceable cartridge and include a processing unit, a display screen, and input functions such as buttons or a keypad. Such pumps may include the ability to run multiple insulin delivery programs, such as basal and bolus programs, to eliminate the need for injections of insulin via needles and syringes, by providing medication via an infusion device that can be worn by the patient for an extended period of time, usually in the range of 1-3 days.

Patients using insulin pumps typically have the ability to program insulin delivery times and amounts into their pump's software, and enter their BG values into the pump via a data input system to deliver boluses of insulin in response to their activities, such as exercise and meal intake. Alternatively, the BG meter and pump may be in communication to permit the meter to transmit the BG reading to the pump along with a recommended bolus value, or to permit the pump or user to determine the appropriate bolus of insulin, if any. While the convenience of an insulin pump may improve the lifestyle of the patient and lessen the imposition of their disease on their normal activity, such persons are still susceptible to experiencing symptoms of diabetes which may render them unable to operate their meter, pump, or both, thereby leaving them unable to self-administer the necessary bolus of insulin in response to abnormal BG levels. As well, when under certain debilitating effects of diabetes, patients may be more prone to incorrectly operating their drug infusion device.

For example, most diabetics that use an insulin infusion device purchase their insulin separately from the cartridges that they insert into their infusion pump. In order to fill the cartridge, they insert a needle attached to the cartridge into an insulin vial and pull back on an extractor. Occasionally, either through inattention, some sort of diminished capacity, or for any of a variety of reasons, the patient or their caregiver may attempt to re-use the drug cartridge. This can present a variety of safety concerns to the patient, as the drug cartridge they insert into their infusion device may not be properly filled or contain the appropriate medication (such as in instances where expired and unexpired medication are used, for example).

Typically, an extractor is supplied as a separate component with the cartridge, in cases where the patient or caregiver does not use pre-filled drug cartridges. The extractor is attached to the plunger to facilitate extraction of the drug from the vial. For safety reasons, once the cartridge is filled, the extractor is discarded after use. Because the extractor is supplied as a separate component, it may be misplaced. The extractor is also formed separate from the plunger, which increases manufacturing costs.

Therefore, it would be desirable for patients and caregivers to have a plunger extractor for drug cartridges that increases safety and ease of use for the patient. Preferably, such a plunger could be a removable plunger extractor that is formed with the plunger during manufacture and that cannot be reused once removed from the plunger.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

FIG. 1A is a perspective view of a plunger having an extractor according to an exemplary embodiment of the invention.

FIG. 1B is a cross sectional view of the plunger illustrated in FIG. 1A.

FIG. 2 is a perspective view of a cartridge barrel and plunger according to an exemplary embodiment of the invention.

FIG. 3 is a perspective view of a drug delivery device that is suitable for use with embodiments of the present invention.

FIGS. 4A - 4D are schematic partial cross-sectional views of a sequence of steps in a process for using the extractor shown in FIGS. 1A and 1B.

### DETAILED DESCRIPTION OF ILLUSTRATIVE EMBODIMENTS OF THE INVENTION

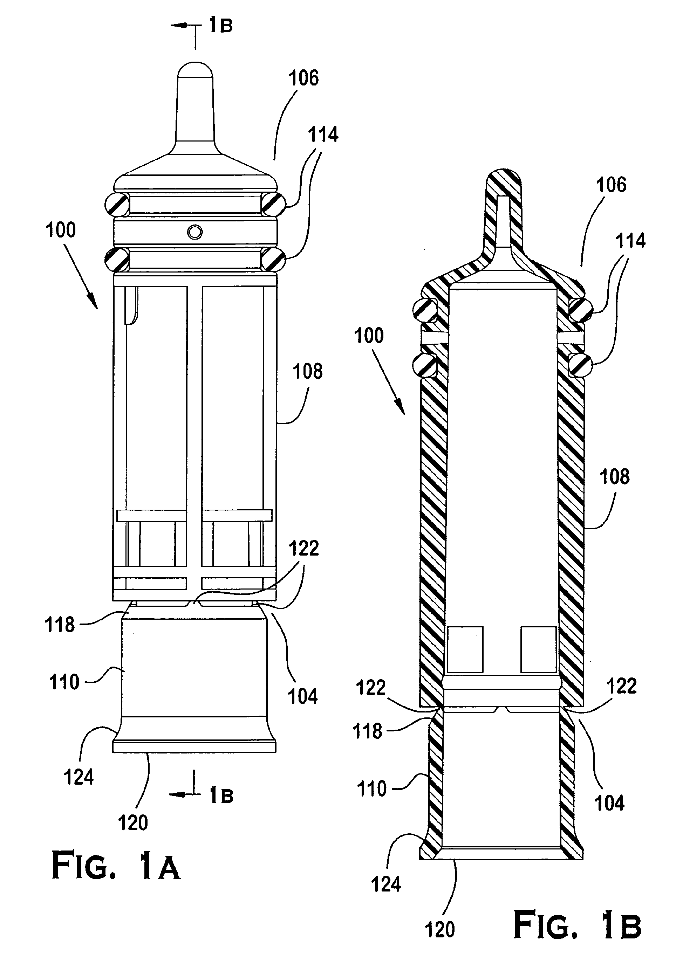

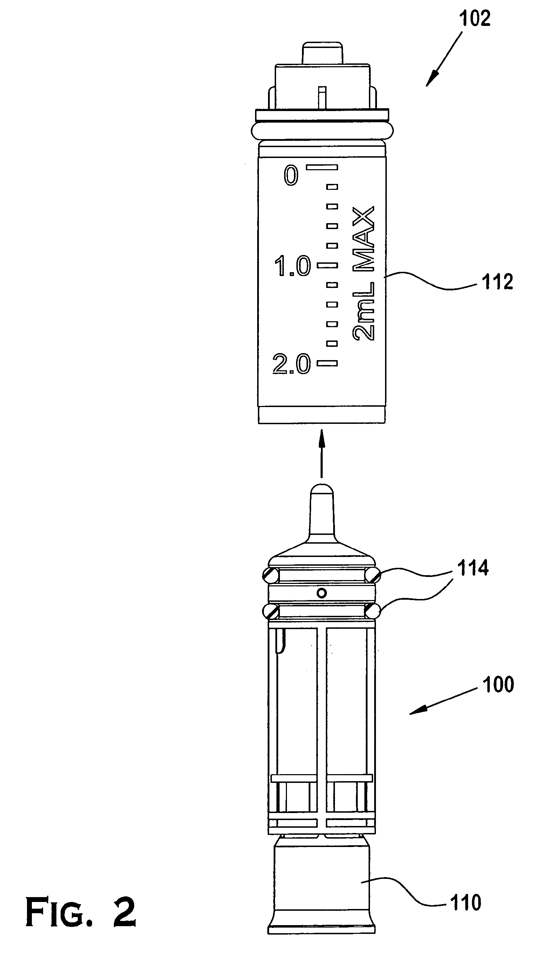

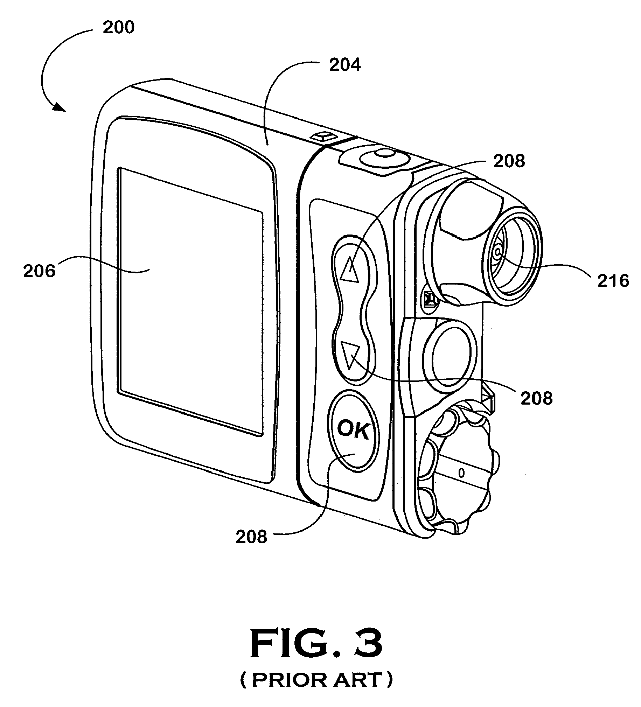

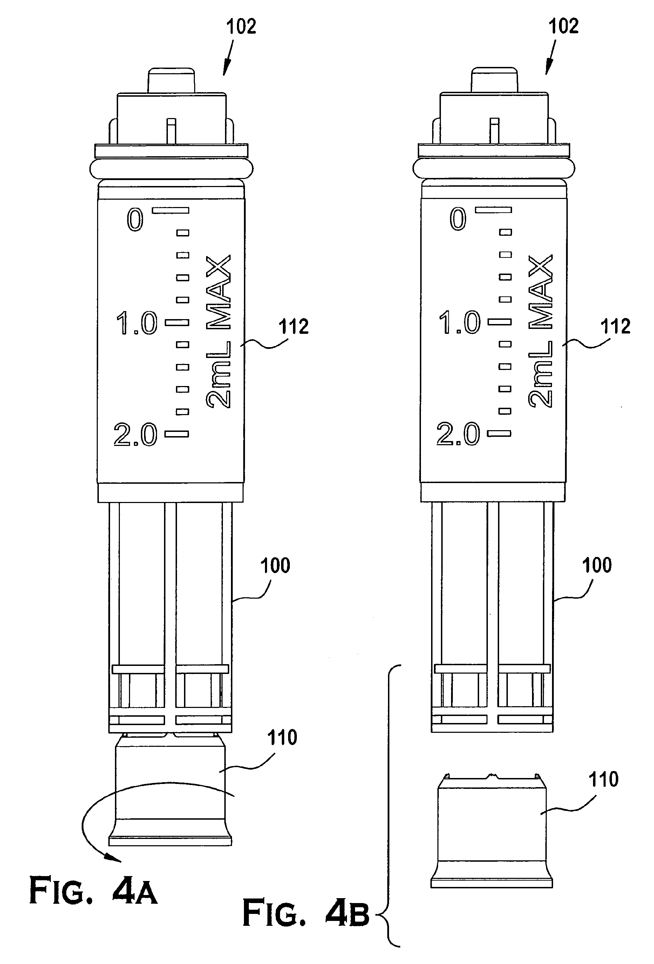

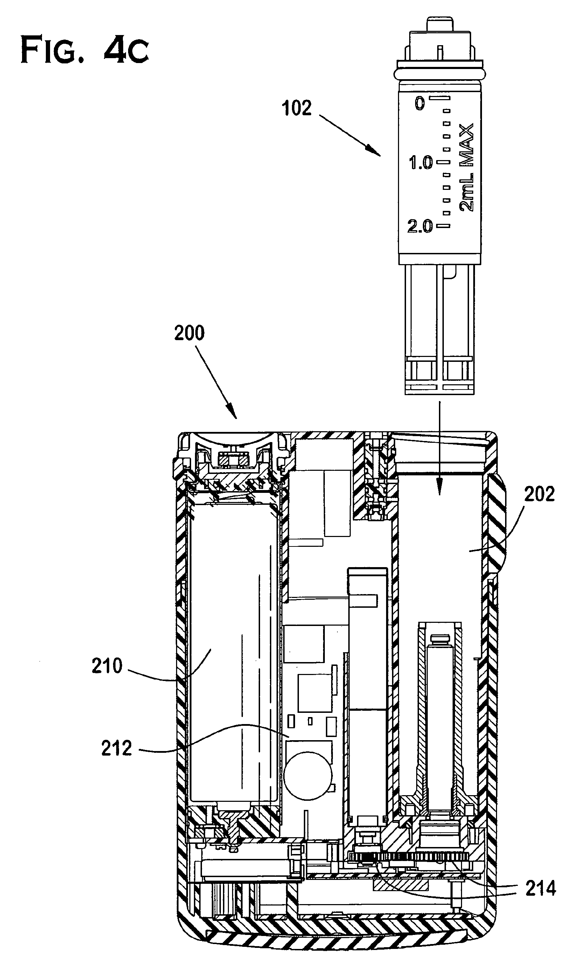

FIGS. 1A and 1B illustrate a plunger 100 for a cartridge 102 configured for receiving and storing a drug according to at least one exemplary embodiment of the present invention. The plunger 100 may generally be cylindrical in shape and include a proximal end 104, a distal end 106, a body 108 and an extractor 110. The extractor may be removably attached to the proximal end 104. The distal end 106 can be slidably inserted into a hollow barrel 112 of the cartridge 102 as illustrated in FIG. 2, and the proximal end 104 of the plunger is slidably inserted into a chamber 202 of a drug delivery device 200. Exemplary illustrations of embodiments of the device are illustrated in FIGS. 3, 4B and 4C.

Referring again to FIGS. 3, 4B and 4C, a typical drug delivery device 200 that may be used with exemplary embodiments of the present invention includes a housing 204, a display 206 for providing operational information to the user, a plurality of navigational buttons 208 for the user to input information, a battery (not shown) in a battery compartment 210 for providing power to drug delivery device 200, processing electronics 212, a drug delivery mechanism 214 (e.g., an insulin pump and drive mechanism) for forcing a drug from a cartridge 102 in a chamber 202 through a side port 216 connected to an infusion set (not shown) and into the body of the user.

FIG. 1A illustrates that the plunger 100 may also include at least one O-ring 114 on distal end 106 that forms a seal between plunger 100 and the inner surface of barrel 112. The O-ring 114 may be a separate component from plunger 100 and may be seated in a groove 116. Alternatively, O-ring 114 may be direct-molded with plunger 100 during manufacture of plunger 100. An advantage of using direct-molded O-ring 114 is that a two-shot molding process can be used which simplifies the plunger assembly process. Another advantage of a direct-molded O-ring 114 is that excess material (i.e., "flash") generated during the molding process will not interfere with the seal between the O-ring 114 and the plunger 100 because the groove 116 is eliminated. The O-ring 114 may be formed of deformable material such as rubber or implantable grade plastic. A direct-molded O-ring 114 may be made of deformable material such as silicone elastomer or other polymeric, plastic, and/or resin materials suitable for industrial molding processes such as injection molding.

The extractor 110 generally has a first end 118 and a second end 120, where the first end 118 of the extractor 110 is connected to the body 108 of the plunger 100 via a plurality of frangible links 122 as shown in FIGS. 1A and 1B. Typically, two or more frangible links 122 connect the first end 118 of the extractor 110 to the body 108. The second end 120 of the extractor 110 includes a flange 124 that can have a larger diameter than that of the chamber 202 in the drug delivery device 200, to prevent insertion of the extractor 110 into the drug delivery device 200. The flange 124 may also facilitates gripping of the extractor 110 by a user during filling of the cartridge 102.

The plunger 100 and the extractor 110 are typically formed from implantable grade plastic, such as long-term implantable plastics including, but not limited to polyethylenes, polyetheretherketones (PEEK) and bioabsorbables-polylactic acid (PLA), polyglycolic acid (PGA) and their copolymers. In instances where the materials do not need to be of an implantable grade, those skilled in the art will readily recognize numerous materials that are suitable for use in industrial molding processes, such as injection molding, including various polyethylene and polyester acrylates and resins.

FIGS. 4A - 4C illustrate an exemplary sequence of steps in a process for using an extractor 110. The provision of an exemplary extractor that may be used with the subject method is depicted in FIGS. 1A and 1B in which like elements of the earlier figures are identified with like numerals.

In a first step of an illustrative method, a drug-filled cartridge 102 is provided in which the cartridge 102 includes a plunger 100 with an extractor 110 on a proximal end 104 of the plunger 100 according to, at least, the exemplary embodiment of FIG. 4A. A drug delivery device 200 (e.g., an insulin pump) is also provided.

As illustrated in FIG. 4B, the extractor 110 can then be removed from the plunger 100 by twisting the extractor 110 to break the frangible links 122. The sheer force required to remove extractor 110 from plunger 100 is between about 0.1 pounds per square inch and about 1.5 pounds per square inch. The compressive load and the tensile load that can be tolerated by the frangible links 122 are generally between about 4 pounds and about 10 pounds per square inch.

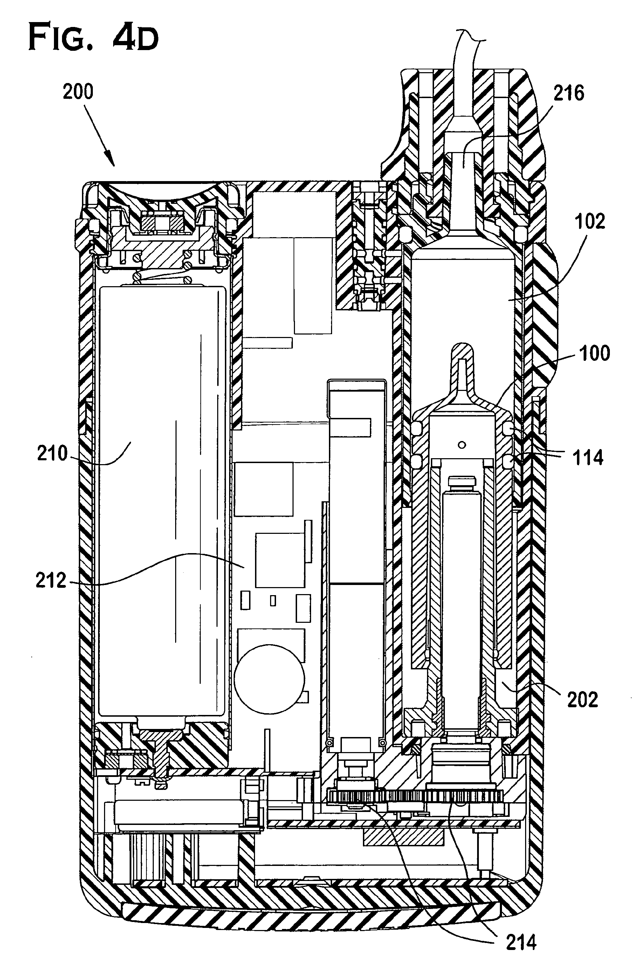

Further, according to the illustrative example, the cartridge 102 is inserted into the chamber 202 of the drug delivery device 200 and is secured therein as illustrated in FIGS. 4C and 4D.

It will be recognized that equivalent structures may be substituted for the structures illustrated and described herein and that the described embodiment of the invention is not the only structure, which may be employed to implement the claimed invention. In addition, it should be understood that every structure described above has a function and such structure can be referred to as a means for performing that function. While embodiments of the present invention have been shown and described herein, it will be obvious to those skilled in the art that such embodiments are provided by way of example only. Numerous variations, changes, and substitutions will now occur to those skilled in the art without departing from the invention.

It should be understood that various alternatives to the embodiments of the invention described herein may be employed in practicing the invention. It is intended that the following claims define the scope of the invention and that methods and structures within the scope of these claims and their equivalents be covered thereby.

## Claims

1. A drug delivery system comprising:
a chamber; and
a cartridge configured for insertion into the chamber,
wherein the cartridge comprises:
a hollow barrel for receiving and storing a drug;
a plunger having a distal end and a proximal end, the distal end of the plunger being slidably insertable and movable within the barrel; and
a breakaway extractor disposed external to the barrel and is removably attached to the proximal end of the plunger.

2. The drug delivery system of claim 1, wherein breakaway extractor comprises at least one flange to prevent insertion of the extractor into the drug delivery device.

3. The drug delivery system of claim 1, wherein at least two frangible links connect the breakaway extractor to the plunger.

4. The drug delivery system of claim 3, wherein the sheer force required to break the frangible links is between about 0.1 pounds per square inch and about 1.5 pounds per square inch.

5. The drug delivery system of claim 3, wherein the frangible links can tolerate a compressive load of between about 4 pounds per square inch and about 10 pounds per square inch.

6. The drug delivery system of claim 3, wherein the frangible links can tolerate a tensile load of between about 4 pounds per square inch and about 10 pounds per square inch.

7. A method of inserting a cartridge into a drug delivery device, the method comprising: providing:
a chamber; and
a cartridge configured for insertion into the chamber,
wherein the cartridge comprises:
a hollow barrel for receiving and storing a drug;
a plunger having a distal end and a proximal end, the distal end of the plunger being slidably insertable and movable within the barrel; and
a breakaway extractor disposed external to the barrel and removably attached to the proximal end of the plunger; filling the cartridge with a drug;
breaking a plurality of frangible links between the plunger and the breakaway extractor
to remove the extractor from the plunger; and
inserting the cartridge into the chamber such that the cartridge is secured therein.
